# EUROPEAN PATENT APPLICATION

(11) **EP 3 961 226 A1**
(43) Date of publication of application: **02.03.2022**
(21) Application number: 20795032.0
(22) Date of filing: 23.04.2020
(51) Int. Cl.: G01N 37/00, G01N 35/08

(54) **FLOW PATH DEVICE, CARTRIDGE, AND MEASUREMENT SYSTEM**

(30) Priority: 25.04.2019 JP 2019084488
(71) Applicant: Kyocera Corporation, Kyoto-shi, Kyoto 612-8501 (JP)
(72) Inventor: IKEDA, Yutaka, Kyoto-shi, Kyoto 612-8501 (JP); MOTOTSU, Kazunori, Kyoto-shi, Kyoto 612-8501 (JP); SUNADAm Takahiro, Kyoto-shi, Kyoto 612-8501 (JP)
(74) Representative: TBK
(86) International application number: PCT/JP2020/017540
(87) International publication number: WO 2020/218439

(57) **Abstract**

A flow path device includes a storage chamber, an inlet, a flow path, and a projection. The storage chamber includes a ceiling surface and a bottom surface, and is capable of storing a specimen. The inlet is one through which an outside of the storage chamber and the storage chamber communicate with each other to allow the specimen to flow into the storage chamber from the outside of the storage chamber. The flow path is connected to an upper portion of the storage chamber at a location situated away from the inlet. The specimen is allowed to flow out into the flow path from the storage chamber. The projection projects toward the bottom surface of the storage chamber from the ceiling surface of the storage chamber, and is positioned between the inlet and the flow path. The projection limits a space with which a space of the storage chamber toward the inlet and a space of the storage chamber toward the flow path communicate to a height less than or equal to a predetermined height from the bottom surface of the storage chamber.

## Description

### CROSS REFERENCES TO RELATED APPLICATIONS

The present application claims priority to Japanese Patent Application No. 2019-084488, filed in Japan on April 25, 2019. The entire disclosure of the earlier application is incorporated herein by reference.

### Technical Field

The present disclosure relates to a flow path device, a cartridge, and a measurement system.

### Background Art

Hitherto, a known testing instrument has included a specimen supply port, a measurement chamber, and a flow path through which the specimen supply port and the measurement chamber communicate with each other (PTL 1).

### Citation List

### Patent Literature

PTL 1: Japanese Unexamined Patent Application Publication No. 2002-224090

### Summary of Invention

A flow path device according to an embodiment of the present disclosure includes
a storage chamber that includes a ceiling surface and a bottom surface, and that is capable of storing a specimen;
an inlet through which an outside of the storage chamber and the storage chamber communicate with each other to allow the specimen to flow into the storage chamber from the outside of the storage chamber;
a flow path that is connected to an upper portion of the storage chamber at a location situated away from the inlet, the specimen being allowed to flow out into the flow path from the storage chamber; and
a projection that projects toward the bottom surface of the storage chamber from the ceiling surface of the storage chamber, and that is positioned between the inlet and the flow path,
wherein the projection limits a space with which a space of the storage chamber toward the inlet and a space of the storage chamber toward the flow path communicate to a height less than or equal to a predetermined height from the bottom surface of the storage chamber.

A cartridge according to an embodiment of the present disclosure includes
the flow path device; and
a sensor part that is connected to the flow path of the flow path device.

A measurement system according to an embodiment of the present disclosure includes
the cartridge; and
an air push-out unit,
wherein the air push-out unit includes
a cylindrical syringe that comprises an end portion insertable into the inlet, and
a plunger that is movable in the syringe and that is capable of pushing out air in the syringe to the end portion.

### Brief Description of Drawings

[Fig. 1] Fig. 1 is a schematic view of a measurement system according to an embodiment of the present disclosure.
[Fig. 2] Fig. 2 is a top view of a cartridge shown in Fig. 1.
[Fig. 3] Fig. 3 is a sectional view of a flow path device along line L-L shown in Fig. 2.
[Fig. 4] Fig. 4 is an exploded view of a part of the flow path device shown in Fig. 3.
[Fig. 5] Fig. 5 is a perspective view of a third base plate shown in Fig. 4.
[Fig. 6] Fig. 6 is a sectional view of a disposed state of the cartridge shown in Fig. 1 in a measurement apparatus.
[Fig. 7] Fig. 7 is a sectional view of another example of a disposed state of the cartridge shown in Fig. 1 in the measurement apparatus.
[Fig. 8] Fig. 8 is a sectional view of a structure when a plunger shown in Fig. 6 has moved downward.
[Fig. 9] Fig. 9 is a sectional view of a structure when the plunger shown in Fig. 8 has moved downward.
[Fig. 10] Fig. 10 is a sectional view of a measurement system according to a comparative example.
[Fig. 11] Fig. 11 is a sectional view of another example of the flow path device shown in Fig. 3. Description of Embodiments

A testing instrument is required to, for example, control a timing of supplying a specimen to a measurement chamber.

The present disclosure relates to providing a flow path device, a cartridge, and a measurement system that are capable of easily controlling a timing of supplying a specimen to a sensor part.

The flow path device, the cartridge, and the measurement system according to an embodiment of the present disclosure are capable of easily controlling a timing of supplying a specimen to the sensor part.

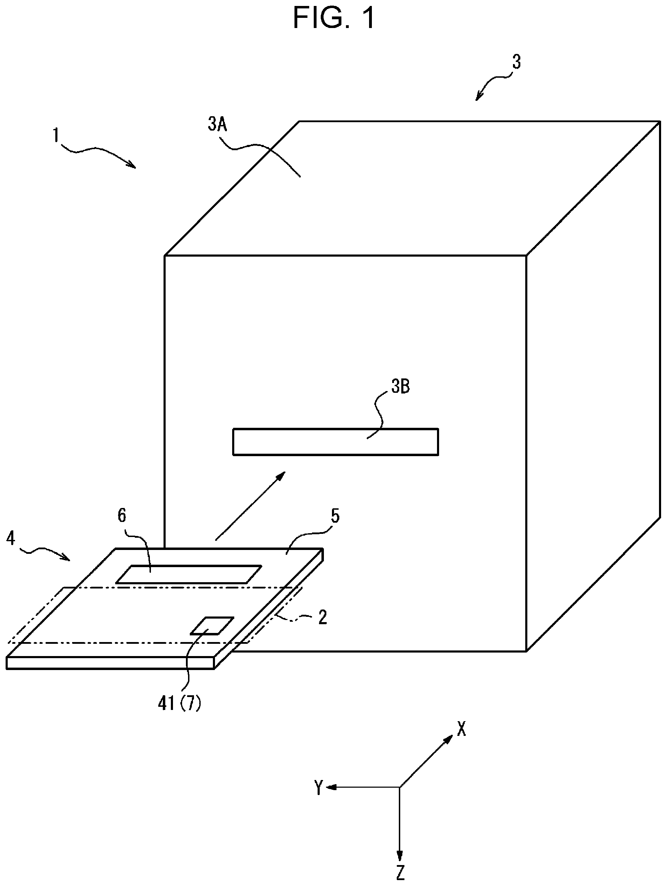

An embodiment according to the present disclosure is described below with reference to the drawings. In the drawings below, the direction of insertion of a flow path device 2 shown in Fig. 1 into a measurement apparatus 3 is a positive direction of an X axis. The direction in which gravity acts is a position direction of a Z axis. A positive direction of a Y axis is determined to form a right-handed orthogonal coordinate system.

Here, in the present disclosure, "down" means the direction in which gravity acts, that is, the positive direction of the Z axis. In the present disclosure, "up" means a direction opposite to the direction in which gravity acts, that is, a negative direction of the Z axis.

### [Structure of Measurement System]

As shown in Fig. 1, a measurement system 1 includes the measurement apparatus 3 and a cartridge 4. The cartridge 4 includes a base plate 5. The cartridge 4 includes the flow path device 2 and a sensor part 6 at the base plate 5. The sensor part 6 may be constituted as a separate member from the base plate 5. The sensor part 6 may be disposed at the measurement apparatus 3.

The flow path device 2 includes a storage chamber 41 described below. The storage chamber 41 stores a specimen 7. Examples of the specimen 7 include a person's blood, a person's cerebrospinal fluid, and a person's urine. Note that before introducing the specimen 7 into the flow path device 2, the specimen 7 may be pre-treated as appropriate.

The measurement apparatus 3 includes a housing 3A and an insertion hole 3B. The housing 3A may be made of, for example, a metal material or a synthetic resin material.

The cartridge 4 is inserted into the measurement apparatus 3 from the insertion hole 3B. By inserting the cartridge 4 from the insertion hole 3B of the measurement apparatus 3, the cartridge 4 is disposed in the measurement apparatus 3. When the cartridge 4 is disposed in the measurement apparatus 3, as described below, the specimen 7 is supplied to the sensor part 6. The sensor part 6 outputs an electric signal in accordance with a component in the specimen 7.

The sensor part 6 may be constituted by, for example, a sensor using a surface acoustic wave (SAW). The measurement apparatus 3 obtains the electric signal output by the sensor part 6. The measurement apparatus 3 measures, for example, the component in the specimen 7 based on, for example, the obtained electric signal.

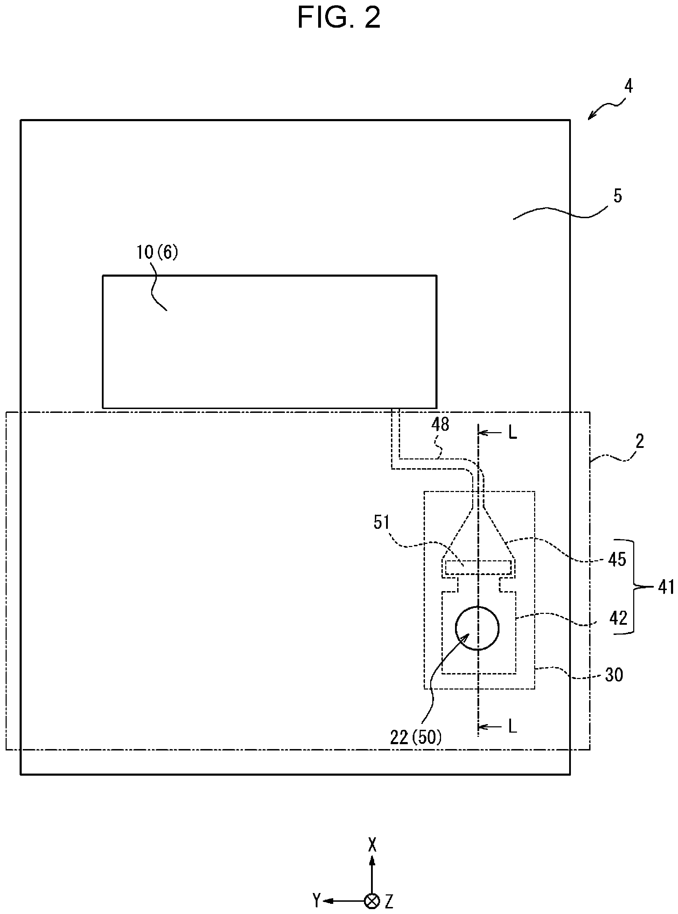

As shown in Fig. 2, the sensor part 6 is disposed in a region 10. At the base plate 5, the region 10 is positioned toward the positive direction of the X axis than the flow path device 2.

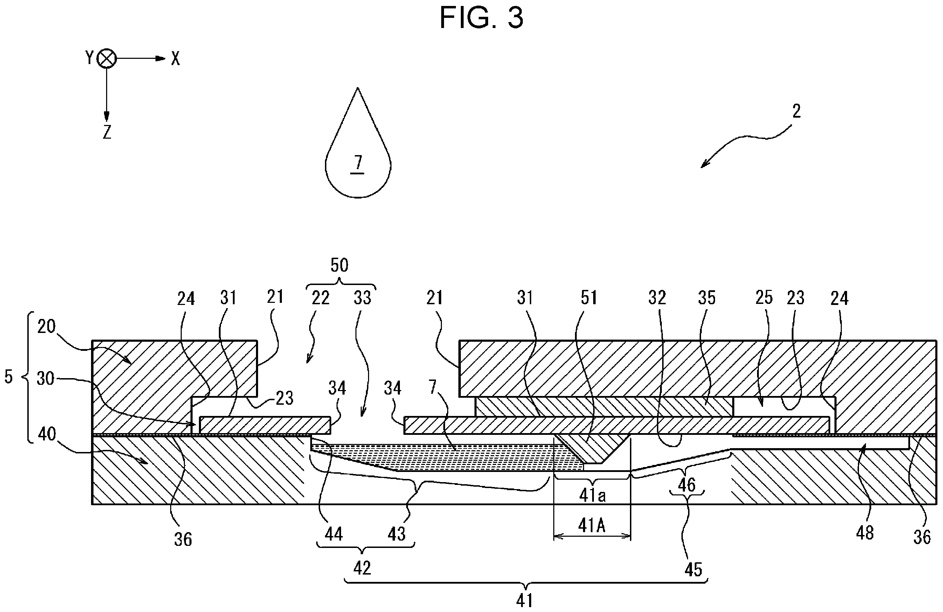

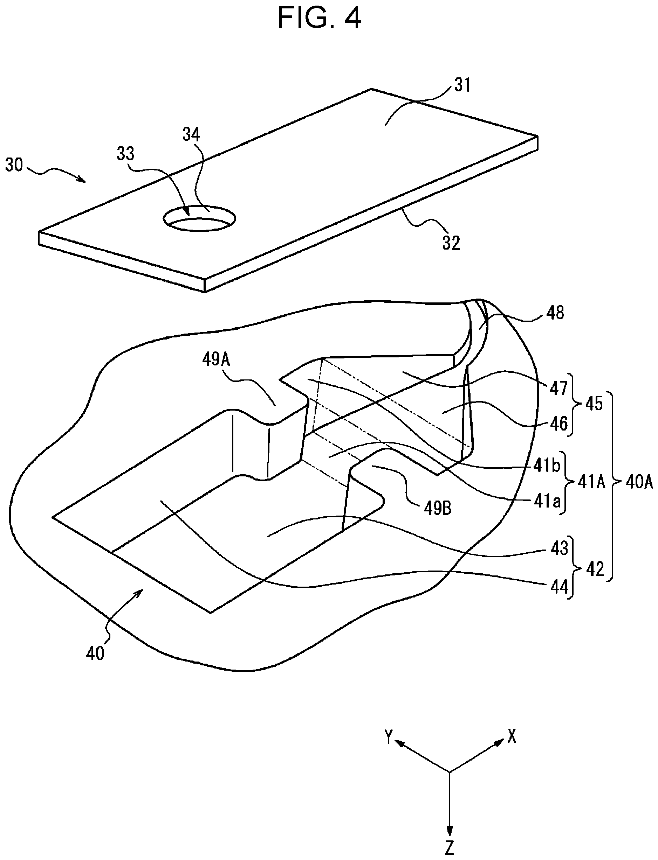

As shown in Fig. 3, the flow path device 2 includes the storage chamber 41, a flow path 48, an inlet 50, and a projection 51. As shown in Fig. 4, the flow path device 2 includes projecting portions 49A and 49B (a pair of projecting portions). As shown in Fig. 3, the base plate 5 includes an upper base plate 20, a small plate 30, a sheet member 36, and a lower base plate 40.

The upper base plate 20 is positioned above the lower base plate 40. The upper base plate 20 may be made of, for example, a synthetic resin material. The upper base plate 20 includes an accommodation portion 25, an inner surface 23 and a wall surface 24 that form the accommodation portion 25, an opening portion 22 that extends through an outer portion of the base plate 5 and the accommodation portion 25, and a wall surface 21 that forms the opening portion 22.

The opening portion 22 is specified as a region that is surrounded by the wall surface 21. As shown in Fig. 2, in top view, the opening portion 22 may be, for example, circular.

The accommodation portion 25 is specified as a region that is surrounded by the inner surface 23 and the wall surface 24. The small plate 30 is accommodated in the accommodation portion 25. The size and the shape of the accommodation portion 25 may be adjusted as appropriate in accordance with the size of the small plate 30.

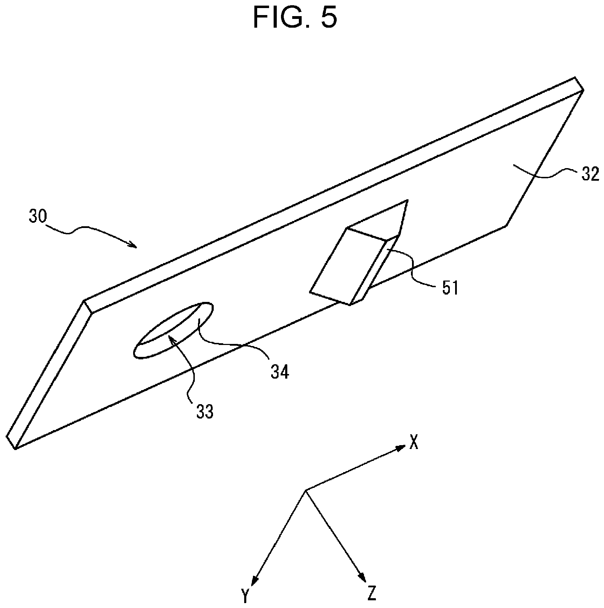

The small plate 30 is positioned between the upper base plate 20 and the lower base plate 40. The small plate 30 is accommodated in the accommodation portion 25 of the upper base plate 20. The small plate 30 may be made of, for example, a synthetic resin material. As shown in Figs. 4 and 5, the small plate 30 has an upper surface 31, a lower surface 32, an opening portion 33 that extends through the upper surface 31 and the lower surface 32, and a wall surface 34 that forms the opening portion 33.

As shown in Fig. 3, the upper surface 31 faces the inner surface 23 of the upper base plate 20. A bonding member 35 may be disposed as appropriate between the upper surface 31 and the inner surface 23 of the upper base plate 20. The bonding member 35 may be any adhesive selected as appropriate in accordance with the material of the small plate 30 and the material of the upper base plate 20. The lower surface 32 faces the lower base plate 40. As shown in Fig. 5, the projection 51 is disposed on the lower surface 32.

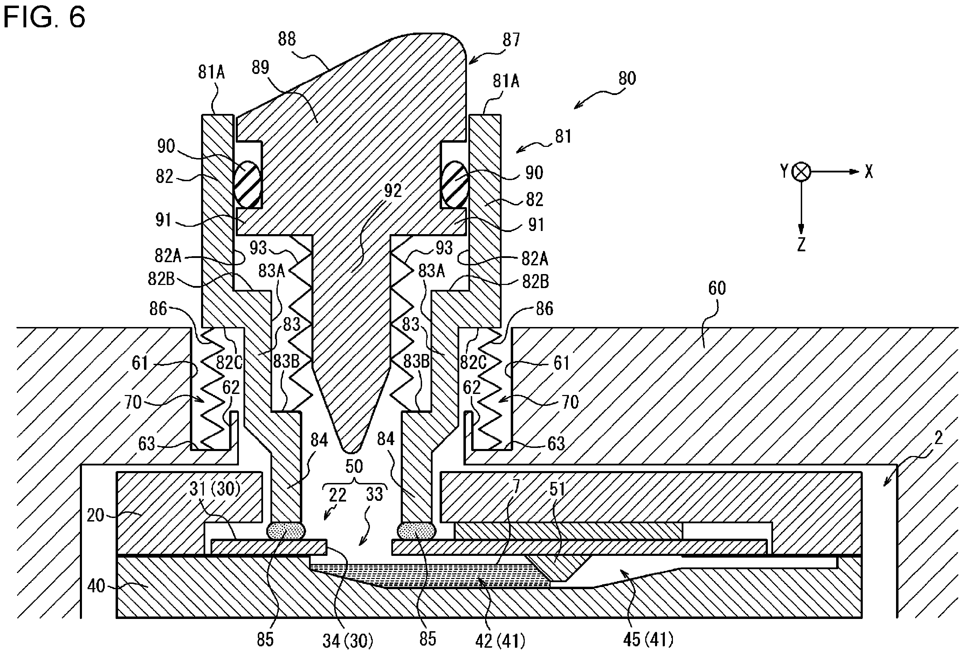

The opening portion 33 is specified as a region that is surrounded by the wall surface 34. As shown in Fig. 4, the opening portion 33 may be circular in top view. As shown in Fig. 3, the inside diameter of the opening portion 33 may be smaller than the inside diameter of the opening portion 22 of the upper base plate 20. The inside diameter of the opening portion 33 may be adjusted as appropriate in accordance with the outside diameter of an end portion 84 of a syringe 81 of an air push-out unit 80 described below (Fig. 6).

As shown in Fig. 3, the sheet member 36 is disposed to cover a gap between the wall surface 24 of the upper base plate 20 and an end portion of the small plate 30 from below the gap. The sheet member 36 may be made of, for example, synthetic resin.

As shown in Fig. 3, the lower base plate 40 is positioned below the upper base plate 20. The lower base plate 40 may be made of, for example, a synthetic resin material. As shown in Fig. 4, the lower base plate 40 may have a recessed portion 40A that opens upward. The recessed portion 40A has a bottom surface 43, a bottom surface 41a, a bottom surface 46, a wall surface 44, a wall surface 41b, and a wall surface 47.

The storage chamber 41 shown in Fig. 3 is specified as a region that is surrounded by the lower surface 32 of the small plate 30 and the surfaces of the recessed portion 40A shown in Fig. 4. For example, the storage chamber 41 is specified as a region that is surrounded by the lower surface 32 of the small plate 30 shown in Fig. 3, the bottom surface 41a, the bottom surface 43, and the bottom surface 46 of the recessed portion 40A shown in Fig. 4, and the wall surface 41b, the wall surface 44, and the wall surface 47 of the recessed portion 40A. Of the lower surface 32 of the small plate 30, a portion that specifies the storage chamber 41 may also be called a "ceiling surface" of the storage chamber 41.

The storage chamber 41 is positioned in the base plate 5. The storage chamber 41 communicates with the outside of the base plate 5 through the inlet 50 including the opening portion 22 and the opening portion 33. In other words, the storage chamber 41 communicates with the outside of the storage chamber 41 through the inlet 50. The storage chamber 41 is capable of storing the specimen 7. For example, as shown in Fig. 3, the specimen 7 dripped toward the inlet 50 by a user is supplied to the storage chamber 41 through the inlet 50. The storage chamber 41 stores the specimen 7 supplied through the inlet 50. The specimen 7 can accumulate at a lower side of the storage chamber 41 due to gravity acting upon the specimen 7. The specimen 7 is stored in the storage chamber 41 until the sensor part 6 shown in Fig. 2 starts to measure the specimen 7. When the sensor part 6 shown in Fig. 2 starts to measure the specimen 7, the specimen 7 stored in the storage chamber 41 is supplied to the sensor part 6 in the region 10 through the flow path 48 shown in Fig. 2.

As shown in Fig. 3, the storage chamber 41 may include a first chamber 42 and a second chamber 45. The storage chamber 41 may have a space 41A between the first chamber 42 and the second chamber 45. As shown in Fig. 4, the space 41A includes a portion that is specified as a region that is surrounded by the bottom surface 41a and the wall surface 41b. As shown in Fig. 4, the first chamber 42 and the second chamber 45 communicate with each other through the space 41A. The projection 51 is positioned in the space 41A. By positioning the projection 51 in the space 41A, as described below, a large amount of the specimen 7 shown in Fig. 3 can be held back at the first chamber 42 in the storage chamber 41.

As shown in Fig. 3, the first chamber 42 is specified as a region that is surrounded by the lower surface 32 of the small plate 30, the bottom surface 43, and the wall surface 44. A part of an upper portion of the first chamber 42 communicates with the inlet 50. As shown in Fig. 4, a part of the first chamber 42 toward the positive direction of the X axis communicates with the second chamber 45 through the space 41A. As shown in Fig. 3, the specimen 7 that has flowed in from the inlet 50 can be stored in the first chamber 42.

As shown in Fig. 4, a side of the bottom surface 43 toward the positive direction of the X axis is continuous with the bottom surface 41a of the space 41A. As shown in Fig. 3, a part of the bottom surface 43 toward the positive direction of the X axis may be a plane surface along an XY plane. As shown in Fig. 3, a part of the bottom surface 43 toward the negative direction of the X axis may be an inclined surface that inclines toward the negative direction of the Z axis as this part extends toward the negative direction of the X axis. As shown in Fig. 4, the wall surface 44 is connected to the wall surface 41b of the space 41A. The bottom surface 43 may have a substantially rectangular shape extending along the XY plane. The wall surface 44 may extend along the negative direction of the Z axis from a part of a periphery of the bottom surface 43. That is, the shape of the first chamber 42 may be a substantially rectangular parallelepiped shape. By forming the first chamber 42 with a substantially rectangular parallelepiped shape, the capacity of the first chamber 42 can be increased. By increasing the capacity of the first chamber 42, even if the specimen 7 is a specimen whose amount is relatively large, such as a person's urine, the first chamber 42 is capable of storing the specimen 7. Therefore, even if the specimen 7 is a specimen whose amount is relatively large, such as a person's urine, the probability with which the specimen 7 overflows the first chamber 42, flows through the second chamber 45, and flows out into the flow path 48 can be reduced. Due to such a structure, the probability with which the specimen 7 stored in the storage chamber 41 reaches the sensor part 6 shown in Fig. 2 through the flow path 48 before the sensor part 6 shown in Fig. 2 starts to measure the specimen 7 can be reduced.

As shown in Fig. 4, the second chamber 45 is specified as a region that is surrounded by the lower surface 32 of the small plate 30, the bottom surface 46, and the wall surface 47. An upper portion of the second chamber 45 toward the positive direction of the X axis communicates with the flow path 48.

As shown in Fig. 4, a side of the bottom surface 46 toward the negative direction of the X axis is continuous with the bottom surface 41a of the space 41A. The wall surface 47 is continuous with the wall surface 41b of the space 41A. The length (width) of the bottom surface 46 in the direction along the Y axis may gradually decrease toward the flow path 48. The wall surface 47 may extend along the negative direction of the Z axis from a part of a periphery of the bottom surface 46. That is, the width of the second chamber 45 may become gradually smaller toward the flow path 48 from a location where the projection 51 is positioned.

As shown in Fig. 3, the bottom surface 46 may be an inclined surface that inclines toward the flow path 48 from the bottom surface 41a of the space 41A. Here, the capacity of the storage chamber 41 can be set based on the quantity of air that is capable of being supplied by the air push-out unit 80 (described later) (Fig. 6). That is, the sum of the capacity of the first chamber 42 and the capacity of the second chamber 45 can be set based on the quantity of air that is capable of being supplied by the air push-out unit 80 (described later) (Fig. 6). In such a case, by causing the bottom surface 46 to be an inclined surface, the capacity of the second chamber 45 is reduced, as a result of which the capacity of the first chamber 42 can be increased by an amount corresponding to the decrease in the capacity of the second chamber 45. By increasing the capacity of the first chamber 42, the probability with which the specimen 7 overflows the first chamber 42, flows through the second chamber 45, and flows out into the flow path 48 can be reduced.

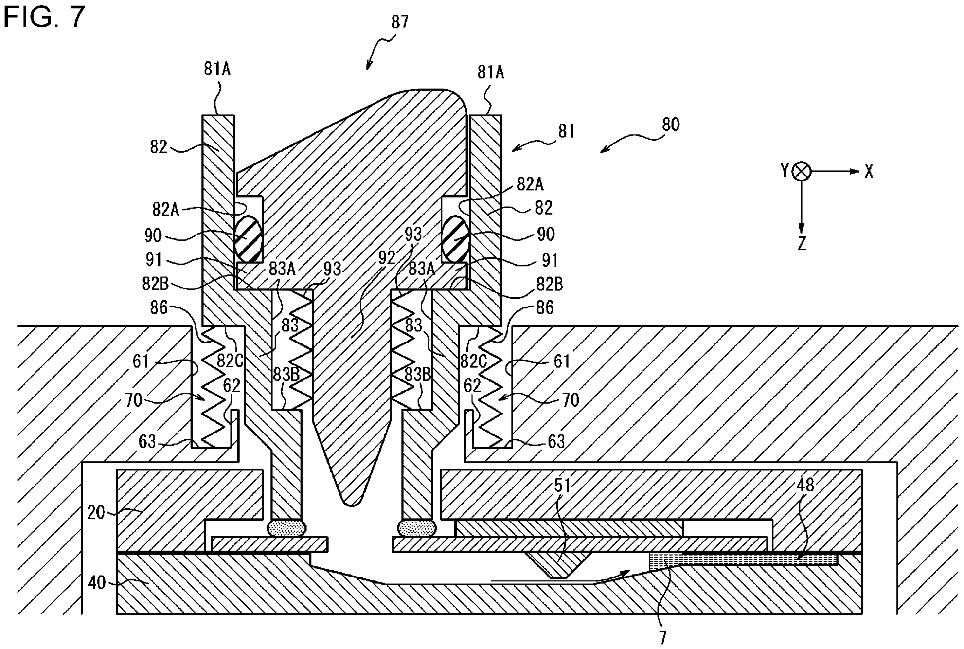

As shown in Fig. 3, the flow path 48 is positioned in the base plate 5. The flow path 48 is specified as a region that is surrounded by a groove in the lower base plate 40 and a lower surface of the upper base plate 20 or the sheet member 36. The flow path 48 is connected to the storage chamber 41 at a location situated away from the inlet 50. For example, in a structure in which the inlet 50 shown in Fig. 3 is connected to the first chamber 42 of the storage chamber 41, the flow path 48 is connected to the second chamber 45 of the storage chamber 41. As shown in Fig. 2, the flow path 48 extends from the storage chamber 41 to the region 10 where the sensor part 6 is disposed. In other words, the sensor part 6 is connected to the flow path 48. The specimen 7 can flow out into the flow path 48 from the storage chamber 41. For example, when the sensor part 6 shown in Fig. 2 starts to measure the specimen 7, air is sent out into the storage chamber 41 by the air push-out unit 80 described below (Fig. 7). As described below, the specimen 7 flows out into the flow path 48 from the storage chamber 41 by the air sent out into the storage chamber 41. The specimen 7 that has flowed out into the flow path 48 reaches the sensor part 6 shown in Fig. 2 through the flow path 48.

The flow path 48 is connected to an upper portion of the storage chamber 41. For example, as shown in Fig. 3, the flow path 48 is connected to the upper portion of the second chamber 45 of the storage chamber 41. Here, the specimen 7 can accumulate at the lower side of the storage chamber 41 due to gravity acting upon the specimen 7. Since the specimen 7 accumulates at the lower side of the storage chamber 41, by connecting the flow path 48 to the upper portion of the storage chamber 41, the probability with which the specimen 7 flows into the flow path 48 before the sensor part 6 shown in Fig. 2 starts to measure the specimen 7 can be reduced. Due to such a structure, the probability with which the specimen 7 reaches the sensor part 6 shown in Fig. 2 through the flow path 48 before the sensor part 6 starts to measure the specimen 7 can be reduced.

As shown in Fig. 4, the projecting portions 49A and 49B are positioned at a portion of the first chamber 42 toward the second chamber 45. The projecting portions 49A and 49B may each be formed as a part of the lower base plate 40.

The projecting portions 49A and 49B face each other. The projecting portion 49A projects toward the negative direction of the Y axis. The projecting portion 449 projects toward the positive direction of the Y axis. By disposing the projecting portions 49A and 49B, it is possible to easily position the projection 51 in the space 41A of the storage chamber 41 in an assembly step of the base plate 5.

As shown in Fig. 3, the inlet 50 has the opening portion 22 of the upper base plate 20 and the opening portion 33 of the small plate 30. That is, the inlet 50 includes a portion that is specified as a region that is surrounded by the wall surface 21 of the upper base plate 20 and a portion that is specified as a region that is surrounded by the wall surface 34 of the small plate 30.

The inlet 50 is positioned in the base plate 5. The inlet 50 causes the outside of the base plate 5 and the storage chamber 41 to communicate with each other to allow the specimen 7 to flow into the storage chamber 41 from the outside of the base plate 5. In other words, the inlet 50 causes the outside of the storage chamber 41 and the storage chamber 41 to communicate with each other to allow the specimen 7 to flow into the storage chamber 41 from the outside of the storage chamber 41. For example, a user drips the specimen 7 toward the inlet 50 from a side of the negative direction of the Z axis. The specimen 7 dripped toward the inlet 50 can flow into the storage chamber 41 from the inlet 50.

As shown in Fig. 3, the projection 51 is positioned in the base plate 5. For example, as shown in Fig. 5, the projection 51 is positioned on the lower surface 32 of the small plate 30. The projection 51 may be integrated with the small plate 30.

As shown in Fig. 3, the projection 51 projects toward the bottom surface 41a of the space 41A of the storage chamber 41 from the lower surface 32 of the small plate 30, which is the ceiling surface of the storage chamber 41. In sectional view along the direction toward the flow path 48 from the inlet 50, the projection 51 may have a substantially inverted triangular shape whose apex is positioned toward the bottom surface 41a of the storage chamber 41.

The projection 51 limits a space with which a space of the storage chamber 41 toward the inlet 50 and a space of the storage chamber 41 toward the flow path 48 communicate to a height less than or equal to a predetermined height from a bottom surface of the storage chamber 41. For example, the projection 51 limits the space 41A with which the first chamber 42 as a space of the storage chamber 41 toward the inlet 50 and the second chamber 45 as a space of the storage chamber 41 toward the flow path 48 communicate to a height less than or equal to the predetermined height from the bottom surface 41a of the storage chamber 41.

Due to the projection 51 limiting the space 41A to a height less than or equal to the predetermined height from the bottom surface 41a of the storage chamber 41, the surface tension of the specimen 7 makes it difficult for the specimen 7 to leak and spread toward the flow path 48 at a location between the projection 51 and the bottom surface 46. By making it difficult for the specimen 7 to leak and spread toward the flow path 48, a large amount of the specimen 7 can be held back at the first chamber 42 in the storage chamber 41. By holding back a large amount of the specimen 7 at the first chamber 42 in the storage chamber 41, the probability with which the specimen 7 flows into the second chamber 45 of the storage chamber 41 before the sensor part 6 shown in Fig. 2 starts to measure the specimen 7 can be reduced. By reducing the probability with which the specimen 7 flows into the second chamber 45 of the storage chamber 41, the probability with which the specimen 7 reaches the sensor part 6 shown in Fig. 2 through the flow path 48 before the sensor part 6 shown in Fig. 2 starts to measure the specimen 7 can be reduced. By holding back a large amount of the specimen 7 at the first chamber 42, a liquid surface of the specimen 7 at the first chamber 42 can be positioned above a lower portion of the projection 51. Due to the liquid surface of the specimen 7 at the first chamber 42 being positioned above the lower portion of the projection 51, as described below, the specimen 7 can be efficiently supplied to the sensor part 6 when the sensor part 6 shown in Fig. 2 starts to measure the specimen 7.

The predetermined height above may be set as appropriate by considering how easily the specimen 7 at the storage chamber 41 leaks and spreads. For example, the predetermined height may be set as appropriate based on the material of the lower base plate 40 and the surface tension of the specimen 7.

Fig. 6 is a sectional view of a disposed state of the cartridge 4 shown in Fig. 1 in the measurement apparatus 3. Fig. 7 is a sectional view of another example of a disposed state of the cartridge 4 shown in Fig. 1 in the measurement apparatus 3. The measurement apparatus 3 shown in Fig. 1 includes a case 60 shown in Fig. 6 and the air push-out unit 80 shown in Fig. 6. The measurement apparatus 3 may include, for example, a heater or a cooler for controlling the temperature of the specimen 7 or the like, and a controller. The controller of the measurement apparatus 3 may be constituted by, for example, a processor or a microcomputer capable of executing application software. The controller of the measurement apparatus 3 may measure, for example, a component in the specimen 7 based on an electric signal output by the sensor part 6.

The case 60 shown in Fig. 6 is disposed in the housing 3A of the measurement apparatus 3 shown in Fig. 1. The case 60 may be made of, for example, a metal material or a synthetic resin material. When the cartridge 4 is inserted from the insertion hole 3B of the measurement apparatus 3 shown in Fig. 1, the flow path device 2 is disposed in the case 60 shown in Fig. 6. The case 60 includes an accommodation portion 70. The accommodation portion 70 is specified as a region that is surrounded by a wall surface 61, a wall surface 62, and a bottom surface 63. The wall surface 61 and the wall surface 62 face each other. One end of the wall surface 61 toward the positive direction of the Z axis and one end of the wall surface 62 toward the positive direction of the Z axis are each continuous with the bottom surface 63. The accommodation portion 70 accommodates a first spring 86 described below.

As shown in Fig. 6, the air push-out unit 80 includes the syringe 81 having an upper surface 81A and a plunger 87 having a contact surface 88. When the flow path device 2 is disposed in the case 60, first, the upper surface 81A of the syringe 81 is pushed downward by the measurement apparatus 3. By pushing the upper surface 81A of the syringe 81 downward, the syringe 81 is pushed downward. Then, the contact surface 88 of the plunger 87 is pushed downward by the measurement apparatus 3. By pushing the contact surface 88 of the plunger 87 downward, the plunger 87 is pushed downward. The structure shown in Fig. 6 is a structure when only the syringe 81 is pushed downward by the measurement apparatus 3. The structure shown in Fig. 7 is a structure when, in addition to the syringe 81, the plunger 87 is pushed downward by the measurement apparatus 3. Note that, when the measurement on the specimen 7 shown in Fig. 1 ends, the measurement apparatus 3 shown in Fig. 1 stops pushing the upper surface 81A of the syringe 81 downward and stops pushing the contact surface 88 of the plunger 87 downward.

As shown in Fig. 6, the syringe 81 has a cylindrical shape. The syringe 81 may be made of, for example, a synthetic resin material or a glass material. The syringe 81 has the upper surface 81A as described above. The syringe 81 further includes a body portion 82, an intermediate portion 83, the end portion 84, a first seal member 85, and the first spring 86. The plunger 87 is movable in the syringe 81, and is capable of pushing out air in the syringe 81 to the end portion 84. The plunger 87 may be made of a synthetic resin material or a glass material. The plunger 87 has the contact surface 88 as described above. The plunger 87 further includes a shaft portion 89, a second seal member 90, a flange portion 91, a rod-like portion 92, and a second spring 93.

The upper surface 81A faces upward. The upper surface 81A can be pushed downward by the measurement apparatus 3 shown in Fig. 1 as described above. By pushing the upper surface 81A downward by the measurement apparatus 3, the syringe 81 can be pushed downward. The upper surface 81A may be the upper surface of the body portion 82.

The body portion 82 may have a cylindrical shape. The body portion 82 may have an inner peripheral surface 82A, a fixing surface 82B, and a lower surface 82C. The inner peripheral surface 82A faces the plunger 87. The fixing surface 82B faces upward. An outer peripheral end of the fixing surface 82B is continuous with a lower portion of the inner peripheral surface 82A. As shown in Fig. 7, when the plunger 87 is pushed downward, the fixing surface 82B abuts upon a lower surface of the flange portion 91 of the plunger 87. Due to the fixing surface 82B abutting upon the lower surface of the flange portion 91 of the plunger 87, the plunger 87 is fixed. As shown in Fig. 6, the lower surface 82C is continuous with an outer peripheral surface of the body portion 82 and an outer peripheral surface of the intermediate portion 83. The lower surface 82C faces downward.

The intermediate portion 83 shown in Fig. 6 may have a cylindrical shape. In a direction along the Z axis, the intermediate portion 83 is positioned between the body portion 82 and the end portion 84. The inside diameter of the intermediate portion 83 is smaller than the inside diameter of the body portion 82. The inside diameter of the intermediate portion 83 is larger than the inside diameter of the end portion 84. The first spring 86 may be disposed around the intermediate portion 83. The intermediate portion 83 has an inner peripheral surface 83A and a fixing surface 83B. The inner peripheral surface 83A faces the plunger 87. An upper portion of the inner peripheral surface 83A is continuous with an inner peripheral end of the fixing surface 82B of the body portion 82. The fixing surface 83B faces upward. An outer peripheral end of the fixing surface 83B is continuous with a lower portion of the inner peripheral surface 83A.

The end portion 84 may have a cylindrical shape. The end portion 84 can be inserted into the inlet 50. For example, when the syringe 81 is pushed downward by the measurement apparatus 3 shown in Fig. 1, as shown in Fig. 6, the end portion 84 is inserted into the inlet 50.

The first seal member 85 is disposed on the end portion 84. The first seal member 85 may be an O ring made of a flexible material, such as rubber. The first seal member 85 may be disposed along an entire lower surface of the end portion 84. The first seal member 85 is capable of hermetically sealing a location between the end portion 84 and the storage chamber 41. For example, when the syringe 81 is pushed downward, as shown in Fig. 6, the first seal member 85 can abut upon a portion of the upper surface 31 of the small plate 30 surrounding the wall surface 34 of the small plate 30. Due to the first seal member 85 abutting upon the portion of the upper surface 31 of the small plate 30 surrounding the wall surface 34 of the small plate 30, the location between the end portion 84 and the storage chamber 41 is hermetically sealed.

The first spring 86 is disposed around the intermediate portion 83. The first spring 86 may be, for example, a coil spring. The first spring 86 may be wound around the outer peripheral surface of the intermediate portion 83. As shown in Fig. 6, when the syringe 81 is pushed downward, the first spring 86 is accommodated in the accommodation portion 70 of the case 60. One end of the first spring 86 is fixed to the lower surface 82C of the body portion 82. The other end of the first spring 86 is a free end. As shown in Fig. 6, when the syringe 81 is pushed downward, the other end of the first spring 86 is pushed against the bottom surface 63 of the accommodation portion 70. By pushing the other end of the first spring 86 against the bottom surface 63 of the accommodation portion 70, the first spring 86 is compressed. When the measurement apparatus 3 stops pushing the upper surface 81A of the syringe 81 downward as a result of ending the measurement on the specimen 7 shown in Fig. 1, the first spring 86 expands. By expanding the first spring 86, an elastic force that moves the syringe 81 upward is applied to the syringe 81. By applying the elastic force to the syringe 81 by the first spring 86, after the measurement apparatus 3 shown in Fig. 1 stops pushing the upper surface 81A of the syringe 81 downward, the syringe 81 moves upward and returns to its original position.

The contact surface 88 is positioned above the shaft portion 89. As described above, the contact surface 88 can be pushed downward by the measurement apparatus 3 shown in Fig. 1. When the contact surface 88 is pushed downward by the measurement apparatus 3 shown in Fig. 1, as shown in Fig. 7, the plunger 87 is pushed downward. When the plunger 87 is pushed downward, as shown in Fig. 7, air in the syringe 81 is sent out into the storage chamber 41. For example, when the plunger 87 is pushed downward, air in a portion of a region surrounded by the inner peripheral surface 82A of the body portion 82 shown in Fig. 6 is sent out into the storage chamber 41, the portion being situated between the fixing surface 82B of the body portion 82 and the lower surface of the flange portion 91. By the air sent out into the storage chamber 41, as described below, the specimen 7 flows into the flow path 48 and is supplied to the sensor part 6 shown in Fig. 2.

The contact surface 88 may be an inclined surface that inclines toward the positive direction of the Z axis with respect to the XY plane. The contact surface 88 can be gradually pushed downward by the measurement apparatus 3 shown in Fig. 1. By gradually pushing the contact surface 88 downward, the plunger 87 is capable of gradually moving downward. By gradually moving the plunger 87 downward, air in the syringe 81 can be gradually sent out into the storage chamber 41.

The shaft portion 89 may have a columnar shape. The contact surface 88 is positioned above the shaft portion 89. The flange portion 91 and the rod-like portion 92 are positioned below the shaft portion 89.

The second seal member 90 is disposed around the shaft portion 89. The second seal member 90 may be an O ring made of a flexible material, such as rubber. The second seal member 90 is capable of hermetically sealing a location between the shaft portion 89 and the inner peripheral surface 82A of the body portion 82. By hermetically sealing the location between the shaft portion 89 and the inner peripheral surface 82A of the body portion 82 by the second seal member 90, the syringe 81 can contain a larger amount of air in the region surrounded by the inner peripheral surface 82A of the body portion 82.

The flange portion 91 is disposed at a lower portion of the shaft portion 89. The flange portion 91 projects toward the inner peripheral surface 82A of the body portion 82 from the shaft portion 89. As shown in Fig. 7, when the plunger 87 is pushed downward, the lower surface of the flange portion 91 abuts upon the fixing surface 82B of the body portion 82.

As shown in Fig. 6, the rod-like portion 92 is positioned at a lower portion of the flange portion 91. The second spring 93 is disposed around the rod-like portion 92. The second spring 93 may be, for example, a coil spring. The second spring 93 may be wound around an outer peripheral surface of the rod-like portion 92. One end of the second spring 93 is fixed to the lower portion of the flange portion 91. The other end of the second spring 93 is fixed to the fixing surface 83B of the intermediate portion 83. As shown in Fig. 7, when the plunger 87 is pushed downward, the distance between the lower surface of the flange portion 91 and the fixing surface 83B of the intermediate portion 83 becomes smaller than the distance between the lower surface of the flange portion 91 and the fixing surface 83B of the intermediate portion 83 shown in Fig. 6. Due to the distance between the lower surface of the flange portion 91 and the fixing surface 83B of the intermediate portion 83 being reduced as a result of the plunger 87 being pushed downward, the second spring 93 is compressed. When the measurement apparatus 3 shown in Fig. 1 stops pushing the contact surface 88 downward as a result of ending the measurement on the specimen 7 shown in Fig. 1, the second spring 93 expands. By expanding the second spring 93, an elastic force that moves the plunger 87 upward is applied to the plunger 87. By applying the elastic force to the plunger 87 by the second spring 93, after the measurement apparatus 3 shown in Fig. 1 stops pushing the contact surface 88 downward, the plunger 87 is pushed upward.

### [Operation of Measurement System]

As shown in Fig. 3, a user drips the specimen 7 toward the inlet 50 of the flow path device 2. The specimen 7 dripped toward the inlet 50 is stored in the storage chamber 41. As described above with reference to Fig. 3, a large amount of the specimen 7 can be held back at the first chamber 42 in the storage chamber 41 by the projection 51. By holding back a large amount of the specimen 7 at the first chamber 42 in the storage chamber 41, the probability with which the specimen 7 flows into the second chamber 45 of the storage chamber 41 can be reduced. In addition, as described above with reference to Fig. 3, by connecting the flow path 48 to the upper portion of the storage chamber 41, the probability with which the specimen 7 accumulated at the lower side of the storage chamber 41 flows into the flow path 48 can be reduced.

After the user has dripped the specimen 7 toward the inlet 50 of the flow path device 2, as shown in Fig. 1, the cartridge 4 including the flow path device 2 is inserted into the insertion hole 3B of the measurement apparatus 3. The cartridge 4 that has been inserted from the insertion hole 3B of the measurement apparatus 3 shown in Fig. 1 is disposed in the case 60 shown in Fig. 6.

When the cartridge 4 is disposed in the case 60 shown in Fig. 6, the sensor part 6 shown in Fig. 2 starts to measure the specimen 7. First, the upper surface 81A of the syringe 81 shown in Fig 6 is pushed downward by the measurement apparatus 3 shown in Fig. 1. When the upper surface 81A of the syringe 81 shown in Fig. 6 is pushed downward, the syringe 81 moves downward. By moving the syringe 81 downward, the end portion 84 of the syringe 81 is inserted into the inlet 50 of the flow path device 2 as shown in Fig. 6.

When the syringe 81 is pushed downward, the contact surface 88 of the plunger 87 of the air push-out unit 80 shown in Fig. 6 is gradually pushed downward by the measurement apparatus 3 shown in Fig. 1. When the contact surface 88 of the plunger 87 is gradually pushed downward, the plunger 87 gradually moves downward.

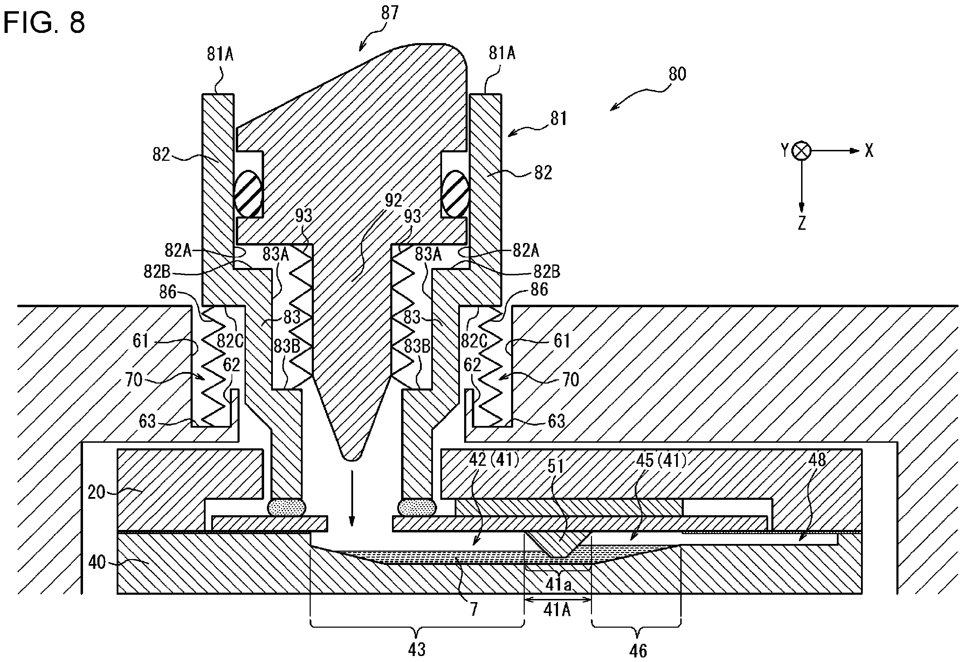

As shown in Fig. 8, when the plunger 87 starts to move downward, as the plunger 87 moves downward, a part of air in the syringe 81 is sent out toward the first chamber 42 of the storage chamber 41. A liquid surface of the specimen 7 stored in the first chamber 42 is pushed downward by the air. When the liquid surface of the specimen 7 in the first chamber 42 is pushed downward, the specimen 7 passes below the projection 51 and flows into the second chamber 42. Due to continued flowing of the specimen 7 into the second chamber 42, the liquid surface of the specimen 7 in the second chamber 45 rises. Due to continued downward movement of the plunger 87, the air continues to push downward the liquid surface of the specimen 7 in the first chamber 42 of the storage chamber 41. Due to the continued downward pushing on the liquid surface of the specimen 7 in the first chamber 42, the liquid surface of the specimen 7 in the second chamber 45 continues to rise. Due to the continued rising of the liquid surface of the specimen 7 in the second chamber 45, the liquid surface reaches the position of the flow path 48.

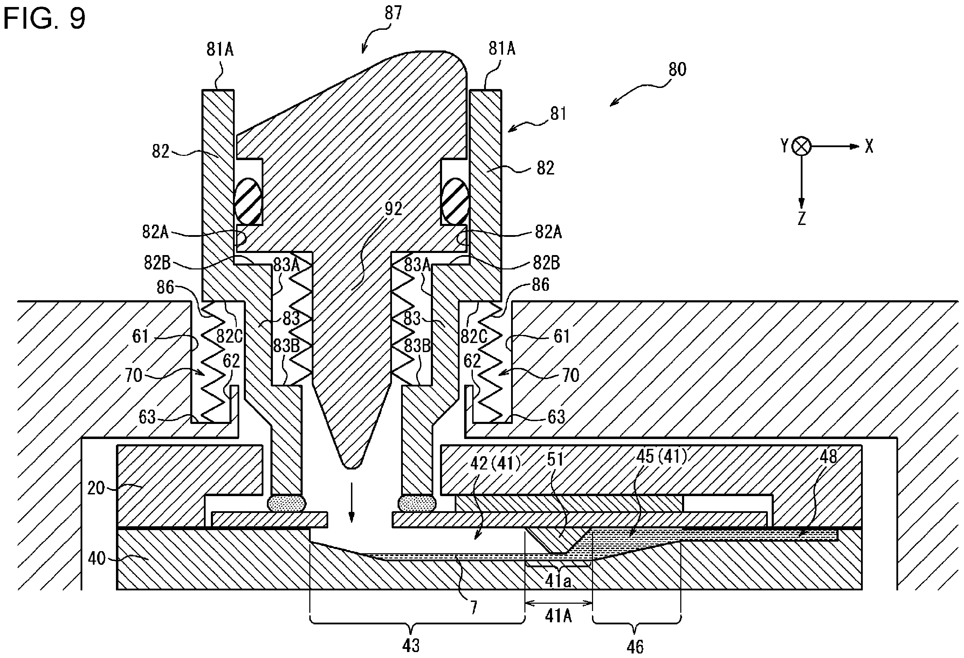

As shown in Fig. 9, when the liquid surface of the specimen 7 in the second chamber 45 reaches the position of the flow path 48, the specimen 7 in the second chamber 45 flows out into the flow path 48. The specimen 7 that has flowed out into the flow path 48 reaches the sensor part 6 shown in Fig. 2 through the flow path 48. The sensor part 6 outputs an electric signal that is in accordance with the component in the specimen 7 to the controller of the measurement apparatus 3 shown in Fig. 1.

Here, as described above, the bottom surface 46 may be an inclined surface that inclines toward the flow path 48 from the bottom surface 41a of the space 41A. Due to the bottom surface 46 being an inclined surface that inclines towards the flow path 48, the specimen 7 can smoothly flow toward the flow path 48 from the second chamber 45.

As shown in Fig. 7, the plunger 87 moves downward until the lower surface of the flange portion 91 of the plunger 87 abuts upon the fixing surface 82B of the syringe 81. In the structure shown in Fig. 7, air from the air push-out unit 80 passes below the projection 51. The air that has passed below the projection 51 pushes out the specimen 7 in the second chamber 45 toward the positive direction of the X axis. Here, as described above, the projection 51 may have in sectional view a substantially inverted triangular shape whose apex is positioned toward the bottom surface 41a of the storage chamber 41. Due to the projection 51 having a substantially inverted triangular shape, air that flows in the upper portion of the first chamber 42 of the storage chamber 41 can be smoothly guided to a location below the projection 51 by an oblique surface of the projection 51. Due to the air being smoothly guided to a location below the projection 51 by the oblique surface of the projection 51, the air is capable of efficiently pushing out the specimen 7 in the second chamber 45 toward the positive direction of the X axis.

When the measurement apparatus 3 shown in Fig. 1 ends the measurement on the specimen 7, the measurement apparatus 3 shown in Fig. 1 stops pushing the upper surface 81A of the syringe 81 shown in Fig. 7 downward and stops pushing the contact surface 88 of the plunger 87 shown in Fig. 7 downward. By stopping the downward pushing on the upper surface 81A of the syringe 81 shown in Fig. 7, as described above, the syringe 81 can be returned to its original position by the first spring 86 shown in Fig. 7. In addition, by stopping the downward pushing on the contact surface 88 of the plunger 87 shown in Fig. 7, the plunger 87 can be returned to its original position by the second spring 93 shown in Fig. 7.

As described above, since the flow path device 2 according to the present embodiment includes the projection 51, as described below, it is possible to easily control a timing of supplying the specimen 7 to the sensor part 6.

### <Comparative Example>

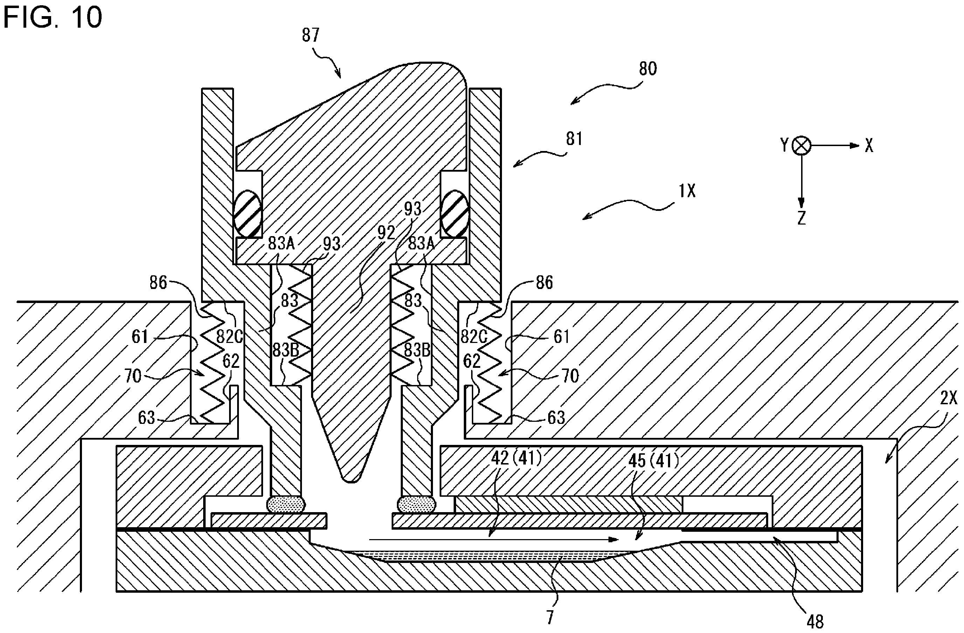

Fig. 10 is a sectional view of a measurement system 1X according to a comparative example. The measurement system 1X includes a flow path device 2X. Unlike the flow path device 2 shown in Fig. 6, the flow path device 2X does not include a projection 51. In such a comparative example, a specimen 7 can spread over the entire bottom surface of a storage chamber 41. Even in the comparative example, similarly to the present embodiment, in order to reduce the probability with which the specimen 7 accumulated at a lower side of the storage chamber 41 flows into a flow path 48, the flow path 48 is connected to an upper portion of the storage chamber 41. In such a measurement system 1X, when air is sent out into the storage chamber 41 by an air push-out unit 80, the air passes the upper portion of the storage chamber 41 and flows into the flow path 48. That is, in the measurement system 1X according to the comparative example, the air from the air push-out unit 80 cannot cause the specimen 7 stored in the storage chamber 41 to flow out into the flow path 48 from the storage chamber 41.

In contrast, in the flow path device 2 shown in Fig. 6, a large amount of the specimen 7 can be held back at the first chamber 42 in the storage chamber 41 by the projection 51. By holding back a large amount of the specimen 7 at the first chamber 42, a liquid surface of the specimen 7 in the first chamber 42 can be positioned above the lower portion of the projection 51. By positioning the liquid surface of the specimen 7 in the first chamber 42 above the lower portion of the projection 51, a space portion of the first chamber 42 above the liquid surface of the specimen 7, excluding the inlet 50, can become a closed space. Due to such a structure, as shown in Fig. 8, air that has flowed in from the inlet 50 can reliably push the liquid surface of the specimen 7 in the first chamber 42 downward. By pushing the liquid surface of the specimen 7 in the first chamber 42 downward, as described above with reference to Fig. 9, the specimen 7 can reach the sensor part 6 shown in Fig. 2 through the flow path 48.

Therefore, according to the present embodiment, it is possible to provide the flow path device 2 and the measurement system 1 that are capable of easily controlling a timing of supplying the specimen 7 to the sensor part 6.

Further, in the measurement system 1 according to the present embodiment, as shown in Fig. 7, it is possible to send out air to the storage chamber 41 by the air push-out unit 80 from above the flow path device 2. Due to such a structure, in the present embodiment, a mechanism for sending out air to the storage chamber 41 does not need to be disposed at the flow path device 2 at a location differing from the location of the storage chamber 41. In the present embodiment, since a mechanism for sending out air to the storage chamber 41 does not need to be disposed at the flow path device 2, it is possible to reduce the size of the flow path device 2 in the XY plane. By reducing the size of the flow path device 2 in the XY plane, it is possible to accommodate a large portion of the flow path device 2 in the measurement apparatus 3 shown in Fig. 1. By accommodating a large portion of the flow path device 2 in the measurement apparatus 3, it is possible to more efficiently increase the temperature of the specimen 7 disposed in the flow path device 2. By more efficiently increasing the temperature of the specimen 7, the measurement system 1 is capable of more precisely measuring, for example, a component of the specimen 7.

In the flow path device 2 according to the present embodiment, as shown in Fig. 3, the lower surface 32 of the small plate 30 can be used as the ceiling surface of the storage chamber 41. The small plate 30 is a component that differs from the lower base plate 40 having the bottom surfaces 43 and 46 and the wall surfaces 44 and 45 of the storage chamber 41. By using the small plate 30 as a component that differs from the lower base plate 40, the storage chamber 41 is less likely to be influenced by surface accuracy. Due to the storage chamber 41 being less likely to be influenced by surface accuracy, it is possible to increase the ability with which the storage chamber 41 is sealed.

The figures for describing the embodiment according to the present disclosure are schematic figures. For example, the dimensional proportions in the figures do not necessarily match the actual dimensional proportions.

Although the embodiment according to the present disclosure has been described based on various figures and examples, it is to be noted that various modifications or corrections can be easily made based on the present disclosure by any person skilled in the art. Therefore, it is to be noted that these modifications or corrections are included within the scope of the present disclosure. For example, the functions or the like of the corresponding structural portions can be rearranged in a noncontradictory manner, a plurality of structural portions or the like can be combined into one or can be separated in a noncontradictory manner.

For example, in the present embodiment above, one end of the first spring 86 shown in Fig. 6 has been described as being fixed to the lower surface 82C of the body portion 82 of the syringe 81. However, the one end of the first spring 86 may not be fixed to the lower surface 82C. The first spring 86 only needs to be disposed around the syringe 81.

For example, in the embodiment above, one end of the second spring 93 shown in Fig. 6 has been described as being fixed to the lower portion of the flange portion 91. The other end of the second spring 93 has been described as being fixed to the fixing surface 83B of the intermediate portion 83. However, the one end of the second spring 93 may not be fixed to the lower portion of the flange portion 91. The other end of the second spring 93 may not be fixed to the fixing surface 83B of the intermediate portion 83. The second spring 93 only needs to be disposed around the rod-like portion 92 of the plunger 87.

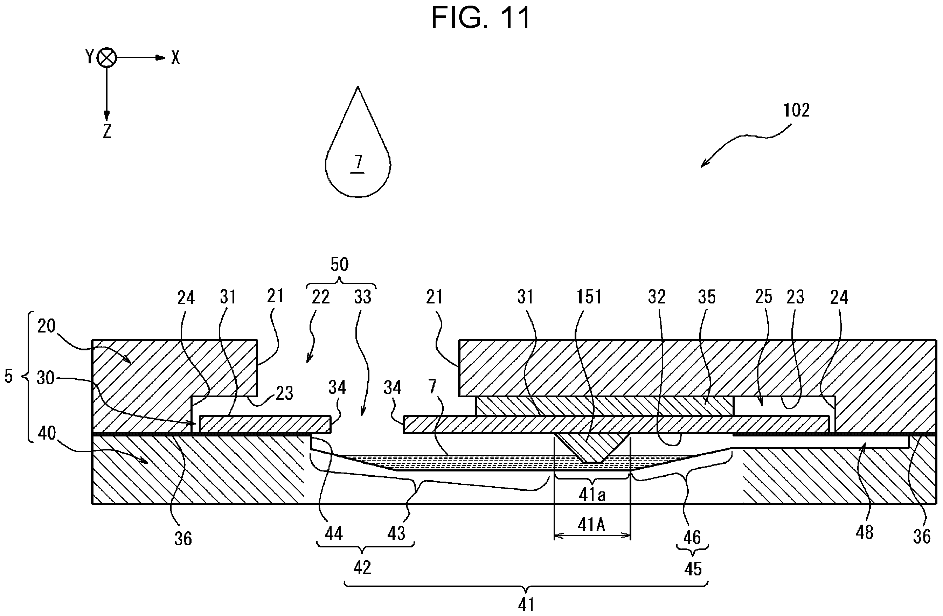

For example, in the present embodiment above, the projection 51 shown in Fig. 3 has been described as limiting the space 41A to a height less than or equal to the predetermined height from the bottom surface 41a of the storage chamber 41. In the description, it has been described that the predetermined height above may be set as appropriate by considering how easily the specimen 7 in the storage chamber 41 leaks and spreads. However, the setting of the predetermined height is not limited thereto. For example, the predetermined height may be set to position the liquid surface of the specimen 7 in the first chamber 42 above the lower portion of the projection 51. A flow path device 102 shown in Fig. 11 includes a projection 151. Similarly to the projection 51 shown in Fig. 3, the projection 151 limits a space 41A to a height less than or equal to a predetermined height from a bottom surface 41a of a storage chamber 41. However, in the structure shown in Fig. 11, the predetermined height is set to position a liquid surface of a specimen 7 in a first chamber 42 above a lower portion of the projection 151. In the structure shown in Fig. 11, the predetermined height may be calculated based on the area of the bottom surface of the entire storage chamber 41 and an assumed amount of the specimen 7. In the structure shown in Fig. 11, the predetermined height is set to position the liquid surface of the specimen 7 in the first chamber 42 above the lower portion of the projection 151. Due to such a structure, in the structure shown in Fig. 11, similarly to the structure shown in Fig. 3, a space portion of the first chamber 42 above the liquid surface of the specimen 7, excluding an inlet 50, can become a closed space. Due to the space portion of the first chamber 42 above the liquid surface of the specimen 7, excluding the inlet 50, becoming a closed space, as described above with reference to Fig. 8, in the flow path device 102, air that has flowed in from the inlet 50 can reliably push the liquid surface of the specimen 7 in the first chamber 42 downward. As described above with reference to Fig. 8, in the flow path device 102, by pushing the liquid surface of the specimen 7 in the first chamber 42 downward, the specimen 7 can reach the sensor part 6 shown in Fig. 2 through the flow path 48.

For example, "first" and "second" in the present disclosure are identifiers for distinguishing between corresponding structures. The structures that are distinguished by, for example, "first" and "second" in the present disclosure can have their numbers exchanged. For example, "the first" in the first chamber, which distinguishes the first chamber from the second chamber, can be replaced by "the second". The identifiers are exchanged at the same time. After the replacement of the identifiers, the structures are distinguished from each other. The identifiers may be deleted. The structures whose identifiers have been deleted are distinguished by their reference signs. Identifiers, such as "first" and "second", in the present disclosure alone should not be used to interpret the order of the corresponding structures or used as a basis for the existence of identifiers containing small numbers.

### Reference Signs List

- 1: measurement system
- 2, 102: flow path device
- 3: measurement apparatus
- 3A: housing
- 3B: insertion hole
- 4: cartridge
- 5: base plate
- 6: sensor part
- 7: fluid
- 10: region
- 20: upper base plate
- 21: wall surface
- 22: opening portion
- 23: inner surface
- 24: wall surface
- 25: accommodation portion
- 30: small plate
- 31: upper surface
- 32: lower surface
- 33: opening portion
- 34: wall surface
- 35: bonding member
- 40: lower base plate
- 41: storage chamber
- 42: first chamber
- 41a, 43, 46: bottom surface
- 41b, 44, 47: wall surface
- 45: second chamber
- 45A: space
- 49A, 49B: projecting portion
- 50: inlet
- 51, 151: projection
- 60: case
- 61, 62: wall surface
- 63: bottom surface
- 70: accommodation portion
- 80: air push-out unit
- 81: syringe
- 81A: upper surface
- 82: body portion
- 82A: inner peripheral surface
- 82B: fixing surface
- 82C: lower surface
- 83: intermediate portion
- 83A: inner peripheral surface
- 83B: fixing surface
- 84: end portion
- 85: first seal member
- 86: first spring
- 87: plunger
- 88: contact surface
- 89: shaft portion
- 90: second seal member
- 91: flange portion
- 92: rod-like portion
- 93: second spring

## Claims

1. A flow path device comprising:
a storage chamber that includes a ceiling surface and a bottom surface, and that is capable of storing a specimen;
an inlet through which an outside of the storage chamber and the storage chamber communicate with each other to allow the specimen to flow into the storage chamber from the outside of the storage chamber;
a flow path that is connected to an upper portion of the storage chamber at a location situated away from the inlet, the specimen allowed to flow out into the flow path from the storage chamber; and
a projection that projects toward the bottom surface of the storage chamber from the ceiling surface of the storage chamber, and that is positioned between the inlet and the flow path,
wherein the projection limits a space with which a space of the storage chamber toward the inlet and a space of the storage chamber toward the flow path communicate to a height less than or equal to a predetermined height from the bottom surface of the storage chamber.

2. The flow path device according to claim 1,
wherein the projection has, in a sectional view along a direction toward the flow path from the inlet, a substantially inverted triangular shape whose apex is positioned toward the bottom surface of the storage chamber.

3. The flow path device according to claim 1 or claim 2, wherein the storage chamber includes
a first chamber that is a space of the storage chamber toward the inlet, and
a second chamber that is a space of the storage chamber toward the flow path.

4. The flow path device according to claim 3,
wherein a bottom surface of the second chamber is an inclined surface that inclines toward the flow path.

5. The flow path device according to claim 3 or claim 4,
wherein the first chamber further includes a plurality of wall surfaces facing each other, the plurality of wall surfaces including a pair of projecting portions that face each other, and
wherein the pair of projecting portions are positioned in a portion of the first chamber that is toward the second chamber.

6. A cartridge comprising:
the flow path device according to any one of claims 1 to 5; and
a sensor part that is connected to the flow path of the flow path device.

7. A measurement system comprising:
the cartridge according to claim 6; and
an air push-out unit,
wherein the air push-out unit includes
a cylindrical syringe that comprises an end portion insertable into the inlet, and
a plunger that is movable in the syringe and that is capable of pushing out air in the syringe to the end portion.

8. The measurement system according to claim 7,
wherein the syringe further comprises a first seal member that is disposed at the end portion and that is capable of hermetically sealing a location between the end portion and the inlet.

9. The measurement system according to claim 7 or claim 8,
wherein the syringe further comprises a first spring that is disposed around the syringe and that applies an elastic force to the syringe, the elastic force moving the syringe upward.

10. The measurement system according to any one of claims 7 to 9,
wherein the plunger further comprises
a shaft portion, and
a second seal member that is disposed around the shaft portion and that is capable of hermetically sealing a location between the shaft portion and an inner peripheral surface of the syringe.

11. The measurement system according to claim 10,
wherein the plunger further comprises
a rod-like portion that is positioned below the shaft portion, and
a second spring that is disposed around the rod-like portion and that applies an elastic force to the plunger, the elastic force moving the plunger upward.
